# EUROPEAN PATENT SPECIFICATION

(11) **EP 0 704 216 B1**
(45) Date of publication and mention of the grant of the patent: **08.12.1999**
(21) Application number: 95111876.9
(22) Date of filing: 28.07.1995
(51) Int. Cl.: A61K 31/725, A61K 9/00, A61K 31/715

(54) **Gel-like pharmaceutical compositions containing chondroitin sulfate salts suitable for oral administration**
Pharmazeutische Zusammensetzung in Form eines Gels mit Chondroitinsulfat in Salzform zur oralen Verabreichung
Compositions pharmaceutiques sous forme de gel contenant des sels de chondroitin sulphate appropriés pour l'administration orale

(30) Priority: 28.09.1994 IT MI941974
(43) Date of publication of application: 03.04.1996
(73) Proprietor: IBSA - INSTITUT BIOCHIMIQUE S.A., CH-6903 Lugano (CH)
(72) Inventor: Nocelli, Luca, I-21016 Luino, (Varese) (IT); Donati, Elisabetta, I-20020 Cavallasca, (Como) (IT); Lualdi, Paolo, I-20020 Grandate, (Como) (IT)
(74) Representative: Gervasi, Gemma, Dr.

(56) References cited:
- WO-A-90/00058
- DATABASE WPI Section Ch, Week 9441 Derwent Publications Ltd., London, GB; Class B07, AN 94-329956 & JP-A-06 256 221 (KIBUN FOOD CHEMIPHAR KK) , 13 September 1994

## Description

### FIELD OF THE INVENTION

The present invention relates to water-soluble pharmaceutical compositions in the form of a gel containing a therapeutically effective amount of a salt of chondroitin sulfate, which consent to obtain rapidly and satisfactorily solutions suitable for oral administration.

### PRIOR ART DISCLOSURE

Chondroitin sulfate belongs to the family of glycosaminoglycans, polymeric molecules of high molecular weight with a disaccharide as a repeating unit, which in living organisms are bound to proteins through polar or covalent bonds, thus giving structures of high biological importance.

Out of the most important glycosaminoglycans are hyarulonic acid, keratan sulfate, heparin, dermatan sulfate and chondroitin sulfate.

Chondroitin sulfate, associated with other glycosaminoglycans occurs in several animal organs and tissues. Its chemical structure is characterized by disaccharidic repeating units, consisting of a variously sulfated β-D-N-acetyl-galactosamine residue linked with a residue of uronic acid (β-D-glucuronic acid), optionally sulfated.

Said endogenous molecule is generally isolated from animal tissues in which it is contained by means of various extraction and purification procedures, and in particular from pork and bovine tracheas and from selachii' cartilaginous skeleton.

Chondroitin sulfate occurs in living organisms mainly in two isomeric forms, differing in the position of the ester sulfate bond on the galactopyranosylaminic residue. Chondroitin sulfate A, or chondroitin 4-sulfate, mainly consists of disaccharide units [(1->4)-O-(β-D-glucopyranosyluronic acid)-(1->3)-O-2-N-acetamido-2-deoxy-β-D-galactopyranosyl-4-sulfate]; chondroitin sulfate C, or chondroitin 6-sulfate, mainly consists of disaccharide units [(1->4)-O-(β-D-glucopyranosyluronic acid)-(1-> 3)-O-2-N-acetamido-2-deoxy-β-D-galactopyranosyl-6-sulfate] (Murata K. and Yokoyama Y., *Anal*. *Biochem*., 149, 261-268, 1985). Non-sulfated as well as di- and tri-sulfated disaccharides have also been identified; their isolation mainly depends on the natural source submitted to extraction and purification procedures and, in particular, on the type of animal, tissue or organ, their age etc.

As used in this specification, the term "chondroitin sulfates" refers to chondroitin 4-sulfate, chondroitin 6-sulfate and mixtures thereof, optionally in presence of other existing forms of chondroitin sulfate known in the state of the art. The preferred chondroitin sulfates are chondroitin 4-sulfate and/or chondroitin 6-sulfate.

Evidences of the therapeutic action of said active principle date back to the middle of the 20th century, when chondroitin sulfate was found to inhibit pepsin proteolytic activity.

Chondroitin sulfate performs several and fundamental functions in the tissues and organs containing same, at physiological conditions: for example, it controls the tissues strength and elasticity and contributes to the organization of cytoskeletal structures. Said active principle is especially used in the therapeutical treatment of several pathological states of the cartilaginous joints, such as inflammatory processes and osteoarthrosis. Furthermore, it exerts a protective action towards cartilaginous tissues and sinovial fluid (Pipitone V.R., *Drugs Exptl*. *Clin*. *Res*., XVII (1) 3-7, 1991; Rovetta G., *Drugs Exptl*. *Clin*. *Res*., XVII (1) 53-57, 1991).

Said active principle is particularly suitable for the treatment of arthrosis, since it is able to protect and restore the articular cartilage affected by degenerative phenomena and since it is a basic component of the same cartilage by its own biochemical nature.

To obtain the desired therapeutic effects, chondroitin sulfate is commonly used in the form of sodium salt, salified at the various acid functions of the disaccharide repeating units, i.e. at the carboxylic functions of the glucuronic acidic moieties and at the sulfate radicals of acetylgalactosamine moieties; it is administered orally in the form of a gel.

According to its polymeric nature, sodium chondroitin sulfate exhibits a low rate of solubility in water and tends to form lumps that hydrate very slowly. Said active principle is mainly administered per os, at daily doses of 400 to 1,600 mg, for periods of months.

Up to now, only three pharmaceutical compositions containing sodium chondroitin sulfate and suitable for oral administration are known in the state of the art, i.e. coated tablets, capsules and granules enlosed in packets to be dissolved in water prior to administration. However, the granular form is only partially soluble in water, especially when it contains high concentrations of the active principle. Therefore, great difficulty is met in dissolving such granular composition in water, since frequently lumps formation takes place. For a complete solubilization of said granules, the solution is to be vigorously stirred for a long time and lumps are to be dissolved by pressing against the walls of the vessel used for dissolving the same formulation.

It follows that it is deeply felt the need for a pharmaceutical composition containing chondroitin sulfate able to provide in very short times aqueous solutions containing this active principle, also in large amounts. This would result in an easier and more agreeable oral administration of chondroitin sulfate, which would positively affect the outcome of therapeutical treatments.

### SUMMARY OF THE INVENTION

The Applicant has now surprisingly found gel-like pharmaceutical compositions containing as the active principle a pharmaceutically effective amount of at least a salt of chondroitin sulfate, in combination with suitable excipients and or diluents.

Said compositions are characterized by a rapid and complete solubility in water, which makes them particularly suitable for oral administration in the treatment of arthrosis.

### DETAILED DESCRIPTION OF THE INVENTION

The characteristics and advantages of the pharmaceutical compositions according to the present invention will be better illustrated in the following detailed description.

The object of the present invention is to provide pharmaceutical compositions in the form of gel, containing as the active principle a pharmaceutically effective amount of at least a suitable organic or inorganic salt of chondroitin sulfate.

Said organic salt is preferably the salt of chondroitin sulfate with amino sugars, such as D-glucosamine and D-galactosamine.

Said inorganic salt is preferably the salt of chondroitin sulfate with ions of alkali or alkaline-earth metals, such as K, Na, Mg or Ca; particularly preferred is the sodium salt, which is easily available on the market.

Said compositions are characterized by a complete solubility in water and are particularly suitable for oral administration.

Per gram of chondroitin sulfate salt, the pharmaceutical compositions according to the present invention contain:
- water, in a quantity ranging from 3 to 20 g and preferably from 4 to 12 g;
- at least a sweetening agent, such as xylitol, sorbitol, maltitol etc., in a quantity ranging from 1 to 5 g, and preferably xylitol in a quantity ranging from 1.4 to 3 g;
- at least a flavouring agent, such as flavour of vanilla, caramel, cocoa etc., in a quantity ranging from 1 to 20 mg, and preferably flavour of vanilla and/or caramel, in a quantity ranging from 5 to 12 mg. Therefore, the gel of the invention, being administered by oral route, can show a pleasant taste; in fact flavouring agents can easily hide the taste of the active principle, which is not particularly strong. Per gram of active ingredient, the pharmaceutical compositions of the invention may optionally contain:
- at least a thickening agent, selected from insoluble products such as microcrystalline cellulose, cross-linked polyvinylpyrrolidone etc., in a quantity ranging from 1 to 50 mg, and preferably microcrystalline cellulose in a quantity ranging from 10 to 25 mg.
   Said agent tends to modulate gel viscosity, which may considerably vary depending on the concentration and molecular weight of chondroitin sulfates. Furthermore, being a natural polymer, in the active principle the molecular weight of chondroitin sulfate may vary from 10,000 to 60,000 dalton.
- At least a preservative, such as sodium benzoate, potassium sorbate, parabens, sodium ascorbate, etc., in a quantity ranging from 1 to 30 mg, and preferably sodium benzoate and/or potassium sorbate, in a quantity ranging from 10 to 25 mg.

The pharmaceutical compositions according to the present invention show a dynamic viscosity ranging from 300 to 4,000 mPa·s, measured at 25°C, using a rotational viscometer with a shear rate of 167 s⁻¹.

The same chondroitin sulfate is able to exert a thickening action in water solutions; it can act as a viscosity promoter, thus yielding a viscous solution that, once mixed with the rest of the aforesaid components at the cited concentrations, becomes a fluid gel, particularly suitable for oral administration in single-dose packet.

The gel according to the present invention may conveniently be prepared by a process comprising the following steps:
A) preparation of an aqueous solution containing a sweetening agent in suitable amounts, by heating at a temperature of 40°-70°C, under vigorous stirring, and following addition of the salt of chondroitin sulfate to said solution, under continuous stirring until complete dissolution. Cooling of the resulting solution to 20°-35°C and final addition of a thickening agent.
B) Separately, preparation of an aqueous mixture containing a preservative and a flavouring agent, at room temperature and under continuous stirring.
C) Addition of the mixture obtained in step (B) to the solution prepared in step (A). The resulting gel is then caused to pass through a sieve having appropriate mesh size in order to remove lumps.

The above preparation procedure is reported only for illustrative and not limitative purposes; the pharmaceutical compositions of the invention may also be prepared according to other procedures known in the state of the art.

The pharmaceutical compositions according to the present invention advantageously consent to obtain aqueous solutions of chondroitin sulfate in real time and containing large amounts of the active principle, as required e.g. in the case of a daily unitary dosage.

In the chondroitin sulfate salts contained in the pharmaceutical compositions of the invention, chondroitin sulfate has preferably a molecular weight ranging from 10,000 to 60,000 dalton; it is preferably used the chondroitin sulfate commonly available on the market, containing 2 to 6 molecules of water per disaccharide unit. Chondroitin sulfate has preferably a sulfates/carboxyls ratio ranging from 0.90 to 1.30 and a sulphur content ranging from 5.5% to 7.5% by weight.

The pharmaceutical compositions according to the present invention can be used in humans for the therapeutical treatment of all the disease states treated by chondroitin sulfate in the state of the art, and in particular in the treatment and prophylaxis of inflammatory processes of the cartilaginous tissues (e.g. osteoarthritis), rheumatoid arthritis and articular inflammations in general, in the chondroprotection of cartilaginous tissues and sinovial fluid, and in hyperlipoproteinaemia.

The pharmaceutical compositions of the invention, orally administrable, preferably contain an amount of chondroitin sulfate salt ranging from 6 to 20 mg/kg/die; a daily unitary dose preferably contains 400 to 1,600 mg of the active principle.

The pharmaceutical compositions of the present invention are preferably administered once or twice a day; however, more frequent administrations may be convenient, at least in some cases, and may vary depending on the patient's conditions.

We report hereinbelow for illustrative but not limitative purposes the following examples of preferred pharmaceutical compositions.

### EXAMPLE 1

### Gel for oral administration, containing 800 mg of sodium chondroitin sulfate

| | |
|---|---|
| Sodium chondroitin sulfate | 800 mg |
| Xylitol | 2,200 mg |
| Sodium benzoate | 6 mg |
| Potassium sorbate | 12 mg |
| Cross-linked microcrystalline cellulose | 8 mg |
| Flavours of vanilla and caramel | 8 mg |
| Demineralized water | q.s. 8,000 mg |

### EXAMPLE 2

### Gel for oral administration, containing 1,200 mg of sodium chondroitin sulfate

| | |
|---|---|
| Sodium chondroitin sulfate | 1,200 mg |
| Xylitol | 1,775 mg |
| Sodium benzoate | 6 mg |
| Potassium sorbate | 12 mg |
| Cross-linked microcrystalline cellulose | 30 mg |
| Flavours of vanilla and caramel | 8 mg |
| Demineralized water | q.s. 8,000 mg |

### EXAMPLE 3

### Preparation of the gel for oral administration, containing sodium chondroitin sulfate, as described in Example 1

A) Purified water (40 kg) was poured into a vessel equipped with stirring and heating systems. Once heating had started, xylitol (22 kg) was added to the solution, under vigorous stirring (25 rpm). Once temperature had reached 60°C, sodium chondroitin sulfate (8.66 kg) with a purity of 92.5% was added to the solution, under continuous vigorous stirring (25 rpm) up to complete dissolution of the active principle. The solution was cooled to approx. 30°C and cross-linked microcrystalline cellulose (80 g) was added to said solution, under stirring at an average shear rate of 15 rpm.
B) Separately, it was prepared a mixture by adding, under stirring at room temperature, in the order: purified water (3 l), sodium benzoate (60 g), potassium sorbate (120 g), flavour of vanilla (40 g) and flavour of caramel (40 g).
C) The flavoured mixture obtained in step (B) was added to the solution obtained in step (A), under continuous stirring at a shear rate of 25 rpm, and the resulting mixture was then added with purified water (6 kg). The pH of the mixture was 5.74.

The resulting gel was caused to pass through a sieve having a mesh size of 1.5 mm, in order to remove possible lumps, thus giving 80 kg of gel for oral administration, having the composition of Example 1. Said gel was easily subdivided into 10,000 doses, each containing 800 mg of sodium chondroitin sulfate.

## Claims

1. A pharmaceutical composition in the form of gel for oral administration, containing as active principle a pharmaceutically effective amount of at least an organic or inorganic salt of chondroitin sulfate and containing, per gram of chondroitin sulfate salt:
- water, in a quantity ranging from 3 to 20 g;
- at least a sweetening agent, in a quantity ranging from 1 to 5 g;
- at least a flavouring agent, in a quantity ranging from 1 to 20 mg;
said composition optionally containing, per gram of chondroitin sulfate salt:
- at least a thickening agent, in a quantity ranging from 1 to 50 mg;
- at least a preservative, in a quantity ranging from 1 to 30 mg.

2. The pharmaceutical composition according to claim 1, characterized in that said chondroitin sulfate has a molecular weight ranging from 10,000 to 60,000 dalton.

3. The pharmaceutical composition according to claim 1, characterized in that said chondroitin sulfate has a sulfates/carboxyls ratio ranging from 0.90 to 1.30 and a sulphur content ranging from 5.5% to 7.5% by weight.

4. The pharmaceutical composition according to claim 1, characterized in that said salt of chondroitin sulfate is the sodium salt.

5. The pharmaceutical composition according to claim 1, characterized in that the quantity of water is ranging from 4 to 12 g.

6. The pharmaceutical composition according to claim 1, characterized in that the sweetening agent is selected among xylitol, sorbitol and maltitol.

7. The pharmaceutical composition according to claim 1, characterized in that said sweetening agent is xylitol, in a quantity ranging from 1.4 to 3 g.

8. The pharmaceutical composition according to claim 1, characterized in that the flavouring agent is selected among vanilla, caramel and cocoa.

9. The pharmaceutical composition according to claim 1, characterized in that the flavouring agent is flavour of vanilla or caramel, in a quantity ranging from 5 to 12 mg.

10. The pharmaceutical composition according to claim 1, characterized in that the thickening agent is selected among microcrystalline cellulose and cross-linked polyvinylpyrrolidone.

11. The pharmaceutical composition according to claim 1, characterized in that said thickening agent is microcrystalline cellulose, in a quantity ranging from 10 to 25 mg.

12. The pharmaceutical composition according to claim 1, characterized in that the preservative is selected among sodium benzoate, potassium sorbate, parabens and sodium ascorbate.

13. The pharmaceutical composition according to claim 1, characterized in that said preservative is sodium benzoate or potassium sorbate in a quantity ranging from 10 to 25 mg.

14. The pharmaceutical composition according to claim 1, characterized in that it has a dynamic viscosity ranging from 300 to 4.000 mPa.s, measured at 25°C, with a shear rate of 167 s⁻¹.

15. The pharmaceutical composition according to claim 1, for use in the treatment and prophylaxis of inflammatory processes of the cartilagenous tissues, rheumatoid arthritis or articular inflammations, in the chondroprotection of cartilagenous tissues and sinovial fluid or in the treatment of hyperlipoproteinaemia.

16. The pharmaceutical composition according to claim 1, for the treatment of arthrosis.

17. The pharmaceutical composition according to claim 1, containing a quantity of chondroitin sulfate salt ranging from 400 to 1,600 mg, in the form of daily unitary doses.

18. A process for preparing a pharmaceutical composition in the form of gel for oral administration, containing as active principle a pharmaceutically effective amount of at least an organic or inorganic salt of chondroitin sulfate, as claimed in claim 1, comprising the following steps:
A) preparing an aqueous solution containing a sweetening agent by heating at a temperature of 40°C-70°C under vigorous stirring, adding the salt of chondroitin sulfate to said solution under continuous stirring until complete dissolution and cooling the resulting solution to 20°C-35°C, optionally followed by final addition of a thickening agent;
B) separately preparing an aqueous mixture containing a preservative and a flavouring agent, at room temperature and under continuous stirring;
C) adding the mixture obtained in step (B) to the solution prepared in step (A) and then causing the resulting gel to pass through a sieve in order to remove lumps.

## Patentansprüche

1. Pharmazeutische Zusammensetzung in Form eines Gels zur oralen Verabreichung, enthaltend als wirksamen Bestandteil eine pharmazeutisch wirksame Menge von wenigstens einem organischen oder anorganischen Chondroitinsulfatsalz und enthaltend pro Gramm Chondroitinsulfatsalz:
- Wasser in einer Menge von 3 bis 20 g
- mindestens einen Süßstoff in einer Menge im Bereich von 1 bis 5 g
- mindestens einen Geschmacksstoff in einer Menge im Bereich von 1 bis 20 mg;
diese Zusammensetzung kann fakultativ pro Gramm Chondroitinsulfatsalz enthalten:
- wenigstens ein Verdickungsmittel in einer Menge im Bereich von 1 bis 50 mg
- mindestens ein Konservierungsmittel in einer Menge im Bereich von 1 bis 30 mg.

2. Pharmazeutische Zusammensetzung gemäß Anspruch 1, dadurch gekennzeichnet, daß das Chondroitinsulfat ein Molekulargewicht von 10.000 bis 60.000 Dalton hat.

3. Pharmazeutische Zusammensetzung gemäß Anspruch 1, dadurch gekennzeichnet, daß Chondroitinsulfat ein Sulfat/Carboxyl-Verhältnis im Bereich von 0,90 bis 1,30 und einen Schwefelgehalt von 5,5 bis 7,5 Gew.% hat.

4. Pharmazeutische Zusammensetzung gemäß Anspruch 1, dadurch gekennzeichnet, daß das Chondroitinsulfatsalz ein Natriumsalz ist.

5. Pharmazeutische Zusammensetzung gemäß Anspruch 1, dadurch gekennzeichnet, daß die Menge des Wassers im Bereich von 4 bis 12 g ist.

6. Pharmazeutische Zusammensetzung gemäß Anspruch 1, dadurch gekennzeichnet, daß der Süßstoff aus Xylitol, Sorbitol und Maltitol ausgewählt ist.

7. Pharmazeutische Zusammensetzung gemäß Anspruch 1, dadurch gekennzeichnet, daß der Süßstoff Xylitol in einer Menge im Bereich von 1,4 bis 3 g ist.

8. Pharmazeutische Zusammensetzung gemäß Anspruch 1, dadurch gekennzeichnet, daß der Geschmacksstoff ausgewählt ist aus Vanille, Karamel und Kakao.

9. Pharmazeutische Zusammensetzung gemäß Anspruch 1, dadurch gekennzeichnet, daß der Geschmacksstoff der Geschmack von Vanille oder Karamel in einer Menge im Bereich von 5 bis 12 mg ist.

10. Pharmazeutische Zusammensetzung gemäß Anspruch 1, dadurch gekennzeichnet, daß das Verdickungsmittel ausgewählt ist aus mikrokristalliner Zellulose und kreuzvernetztem Polyvinylpyrrolidon.

11. Pharmazeutische Zusammensetzung gemäß Anspruch 1, dadurch gekennzeichnet, daß das Verdickungsmittel mikrokristalline Zellulose in einer Menge im Bereich von 10 bis 25 mg ist.

12. Pharmazeutische Zusammensetzung gemäß Anspruch 1, dadurch gekennzeichnet, daß das Konservierungsmittel ausgewählt ist aus Natriumbenzoat, Kaliumsorbat, Paraben und Natriumascorbat.

13. Pharmazeutische Zusammensetzung gemäß Anspruch 1, dadurch gekennzeichnet, daß das Konservierungsmittel Natriumbenzoat oder Kaliumsorbat in einer Menge im Bereich von 10 bis 25 mg ist.

14. Pharmazeutische Zusammensetzung gemäß Anspruch 1, dadurch gekennzeichnet, daß sie eine dynamische Viskosität im Bereich von 300 bis 4.000 mPa·s hat, gemessen bei 25°C, mit einer Scherrate von 167 s⁻¹.

15. Pharmazeutische Zusammensetzung gemäß Anspruch 1 zur Verwendung in der Behandlung oder Prophylaxe von entzündlichen Prozessen von verknorpeltem Gewebe, rheumatoider Arthritis oder artikulären Entzündungen, bei der Chondroprotektion von verknorpelten Geweben und Sinovialflüssigkeit oder bei der Behandlung von Hyperlipoproteinämie.

16. Pharmazeutische Zusammensetzung gemäß Anspruch 1 zur Behandlung von Arthrose.

17. Pharmazeutische Zusammensetzung gemäß Anspruch 1, enthaltend eine Menge von Chondroitinsulfatsalz im Bereich von 400 bis 1.600 mg in Form einer täglichen Einheitsdosis.

18. Verfahren zur Herstellung der pharmazeutischen Zusammensetzung in der Form eines Gels zur oralen Verabreichung, enthaltend als wirksamen Bestandteil eine pharmazeutisch wirksame Menge von wenigstens einem organischen oder anorganischen Chondroitinsulfatsalz, wie im Anspruch 1 beansprucht, umfassend die folgenden Schritte:
A) Herstellung einer wäßrigen Lösung enthaltend einen Süßstoff durch Erhitzen auf eine Temperatur von 40°C-70°C unter starken Rühren, Zugabe des Chondroitinsulfatsalzes zu dieser Lösung unter ständigem Rühren bis zur vollständigen Auflösung und Abkühlen der erhaltenen Lösung auf 20°C-35°C, fakultativ gefolgt von einer abschließenden Zugabe eines Verdickungsmittels;
B) getrenntes Herstellen einer wäßrigen Mischung enthaltend ein Konservierungsmittel und einen Geschmacksstoff bei Raumtemperatur unter ständigem Rühren;
C) Zugabe der Mischung erhalten im Schritt (B) zu der Lösung hergestellt im Schritt (A) und anschließend Durchleiten des erhaltenen Gels durch ein Sieb, um Klumpen zu entfernen.

## Revendications

1. Composition pharmaceutique sous la forme de gel pour l'administration orale, contenant en tant que composant actif une quantité efficace du point de vue pharmaceutique d'au moins un sel organique ou inorganique de sulfate de chondroïtine et contenant, par gramme de sel de sulfate de chondroïtine ;
- de l'eau, en une quantité comprise dans la gamme de 3 à 20 g ;
- au moins un agent édulcorant, en une quantité comprise dans la gamme de 1 à 5 g ;
- au moins un parfum, en une quantité comprise dans la gamme de 1 à 20 mg ;
ladite composition contenant éventuellement, par gramme de sel de sulfate de chondroïtine :
- au moins un agent épaississant, en une quantité comprise dans la gamme de 1 à 50 mg ;
- au moins un conservateur, en une quantité comprise dans la gamme de 1 à 30 mg.

2. Composition pharmaceutique suivant la revendication 1, caractérisée en ce que ledit sulfate de chondroïtine a un poids moléculaire compris dans la gamme de 10 000 à 60 000 dalton.

3. Composition pharmaceutique suivant la revendication 1, caractérisée en ce que ledit sulfate de chondroïtine a un rapport sulfate/carboxy compris dans la gamme de 0,90 à 1,30 et une teneur en soufre comprise dans la gamme de 5,5% à 7,5% en poids.

4. Composition pharmaceutique suivant la revendication 1, caractérisée en ce que ledit sel de sulfate de chondroïtine est le sel de sodium.

5. Composition pharmaceutique suivant la revendication 1, caractérisée en ce que la quantité d'eau est comprise dans la gamme de 4 à 12 g.

6. Composition pharmaceutique suivant la revendication 1, caractérisée en ce que l'agent édulcorant est choisi parmi le xylitol, le sorbitol et le maltitol.

7. Composition pharmaceutique suivant la revendication 1, caractérisée en ce que ledit agent édulcorant est le xylitol, en une quantité comprise dans la gamme de 1,4 à 3 g.

8. Composition pharmaceutique suivant la revendication 1, caractérisée en ce que le parfum est choisi parmi la vanille, le caramel et le cacao.

9. Composition pharmaceutique suivant la revendication 1, caractérisée en ce que le parfum est un arôme de vanille ou du caramel, en une quantité comprise dans la gamme de 5 à 12 mg.

10. Composition pharmaceutique suivant la revendication 1, caractérisée en ce que l'agent épaississant est choisi parmi une cellulose microcristalline et une polyvinylpyrrolidone réticulée.

11. Composition pharmaceutique suivant la revendication 1, caractérisée en ce que ledit agent épaississant est une cellulose microcristalline, en une quantité comprise dans la gamme de 10 à 25 mg.

12. Composition pharmaceutique suivant la revendication 1, caractérisée en ce que le conservateur est choisi parmi le benzoate de sodium, le sorbate de potassium, les parabens et l'ascorbate de sodium.

13. Composition pharmaceutique suivant la revendication 1, caractérisée en ce que ledit conservateur est le benzoate de sodium ou le sorbate de potassium en une quantité comprise dans la gamme de 10 à 25 mg.

14. Composition pharmaceutique suivant la revendication 1, caractérisée en ce qu'elle a une viscosité dynamique comprise dans la gamme de 300 à 4000 mPa.s, mesurée à 25°C, avec un taux de cisaillement de 167 s⁻¹.

15. Composition pharmaceutique suivant la revendication 1, utile dans le traitement et la prophylaxie de processus inflammatoires des tissus cartilagineux, de la polyarthrite rhumatoïde ou des inflammations articulaires, dans la chondroprotection de tissus cartilagineux et de fluide sinovial ou dans le traitement de l'hyperlipoprotéinémie.

16. Composition pharmaceutique suivant la revendication 1, pour le traitement de l'arthrose.

17. Composition pharmaceutique suivant la revendication 1, contenant une quantité de sel de sulfate de chondroïtine comprise dans la gamme de 400 à 1 600 mg, sous la forme de doses unitaires quotidiennes.

18. Procédé pour préparer une composition pharmaceutique sous la forme de gel pour l'administration orale, contenant en tant que composant actif une quantité efficace du point de vue pharmaceutique d'au moins un sel organique ou inorganique de sulfate de chondroïtine, suivant la revendication 1, comprenant les étapes suivantes :
(A) préparation d'une solution aqueuse contenant un agent édulcorant par chauffage à une température de 40°C à 70°C sous vigoureuse agitation, addition du sel de sulfate de chondroïtine à ladite solution tandis que l'agitation est poursuivie jusqu'à dissolution complète et refroidissement de la solution résultante à une température de 20°C à 35°C, éventuellement suivi d'une addition finale d'un agent épaississant ;
(B) préparation séparée d'un mélange aqueux contenant un conservateur et un parfum, à la température ambiante et sous agitation constante ;
(C) addition du mélange obtenu dans l'étape (B) à la solution préparée dans l'étape (A) et ensuite envoi du gel résultant à travers un tamis pour éliminer les grumeaux.
